# EUROPEAN PATENT APPLICATION

(11) **EP 1 997 906 A1**
(43) Date of publication of application: **03.12.2008**
(21) Application number: 07109438.7
(22) Date of filing: 01.06.2007
(51) Int. Cl.: C12Q 1/68

(54) **Lactobacillus**

(71) Applicant: Friesland Brands B.V., 7943 PE Meppel (NL)
(72) Inventor: Nauta, Arjen, 6721 CG Bennekom (NL); van den Heuvel, Elisabeth Gertruda Hendrika Maria, 3572 GS Utrecht (NL); Veurink, Janine Henriët, 7681 SK Vroomshoop (NL); Geurts, Johannes Marie Wilhelmus, 7559 NN Hengelo (NL)
(74) Representative: van Loon, C.J.J.

(57) **Abstract**

The invention provides means and methods for detecting, amplifying, quantifying and/or isolating nucleic acid of at least one *Lactobacillus* organism.

## Description

The invention relates to the fields of biology and microbiology.

*Lactobacillus* is a genus of Gram-positive facultative anaerobic bacteria. The genus Lactobacillus is classified into at least 50 (sub-)species, of which several inhabit the human intestinal lumen. Lactobacilli have health-promoting properties since they improve food uptake in the gastrointestinal tract and they are associated with resistance to gastroenterological infections and resistance to tumors such as colon cancer. They are also associated with a lower incidence of allergies.

The presence of Lactobacilli in the gastrointestinal tract is important for the well-being of individuals. A decrease of the amount of Lactobacilli, for instance resulting from immune depression, disease, stress or the use of antibiotics, other drugs or excess alcohol, adversely influences an individual's well-being. A diminished amount of Lactobacilli has an influence on the general health status and often coincides with an increase in susceptibility to gastroenterological infections and chronic diseases like colon cancer.

A sufficiently high amount of Lactobacilli is for instance obtained and/or maintained by the uptake of probiotics and/or prebiotics. Probiotics and prebiotics are useful for improving and/or restoring a normal healthy amount of Lactobacilli in an individual's digestive tract. Probiotics are live micro-organisms, such as *Lactobacillus,* that are capable of improving the balance of the intestinal microflora. Probiotic bacterial cultures assist the body's naturally occurring flora within the digestive tract to reestablish themselves. Prebiotics are food ingredients that beneficially affect the host by selectively stimulating the growth and/or the activity of a limited number of bacteria in the colon.

Lactobacilli belong to the group of the lactic acid bacteria which is named as such because most of its members produce lactic acid. Lactic acid bacteria lower the carbohydrate contents of the foods they ferment, as well as the pH due to lactic acid formation. The pH often drops low enough to inhibit the growth of some harmful pathogens. Lactic acid bacteria play an important role in the production of dairy products. Widely used milk fermenting cultures are for instance *Lactobacillus delbrueckii* subspecies *bulgaricus* which, together with *Streptococcus thermophilus,* form the commonly used yoghurt producing culture. *Lactobacillus lactis* is also used as a starter culture, for instance for producing the Scandinavian dairy products viili and longfil.

The composition of complex microbial populations such as for instance present in biological samples and food samples is often investigated, for instance during health control and food quality control. In various applications the presence, identity and/or amount of Lactobacilli is investigated. The presence and/or amount of Lactobacilli in a sample is often determined using a *Lactobacillus-specific* probe. Nucleic acid present in a sample is often amplified using *Lactobacillus-specific* primers, after which the presence of *Lactobacillus-*specific nucleic acid is detected. During such detection methods, false positive results and false negative results are preferably avoided. False positive results are obtained if nucleic acid other than *Lactobacillus* nucleic acid is detected. False negative results are obtained if nucleic acid of one or more strains of *Lactobacillus* present in a sample is not detected. Currently used nucleic acid detection methods are often not specific enough (yielding false-positive results) or too specific, so that nucleic acid of one or more *Lactobacillus* strains are not detected (yielding false-negative results). An example of a currently used method is disclosed in Song et al. (FEMS Microbiology Letters 187 (2000) 167-173). With the primers used by Song et al. *non-Lactobacillus* nucleic acid is amplified as well. Hence, improvement of the specificity of these primers is desired.

It is an object of the present invention to provide means and methods for detecting, amplifying, identifying and/or isolating *Lactobacillus* nucleic acid.

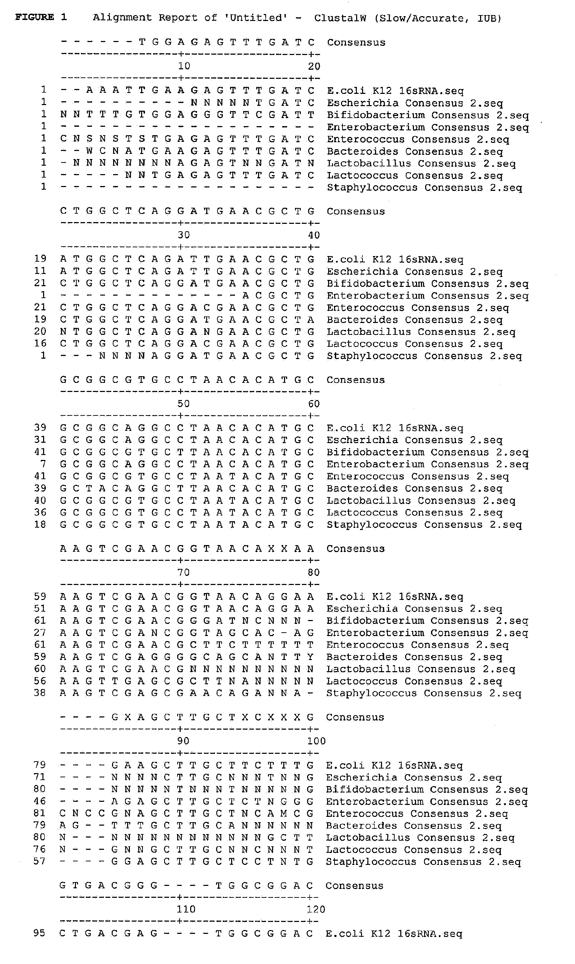

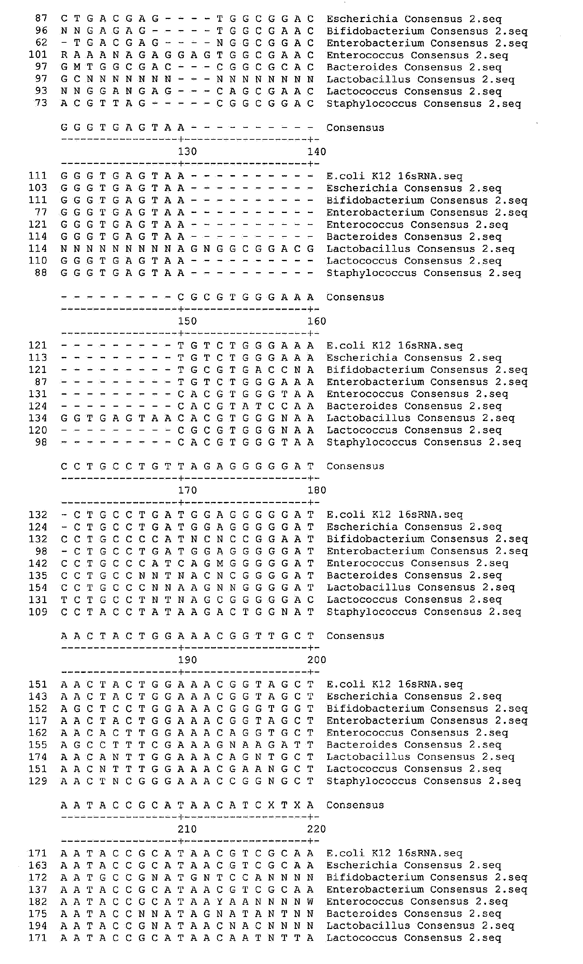

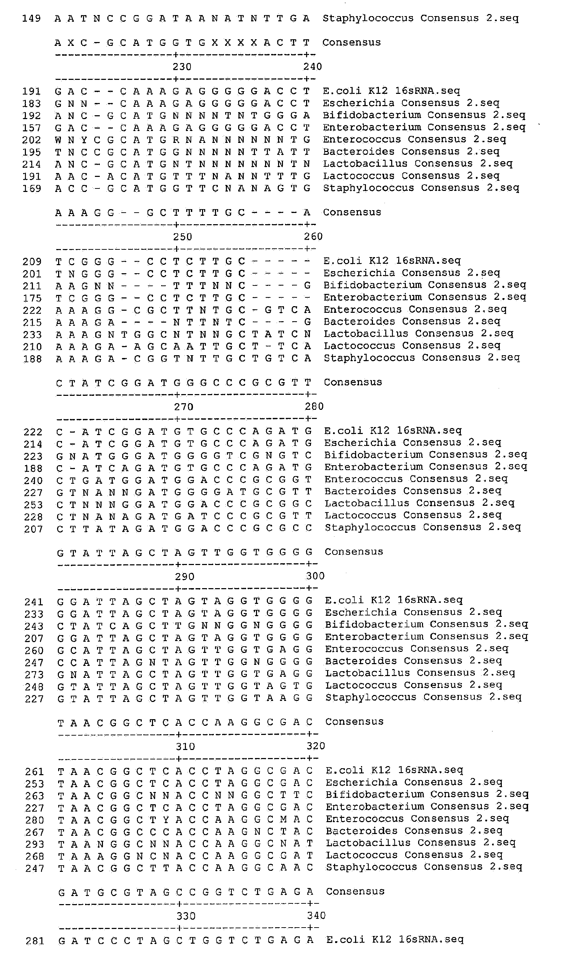

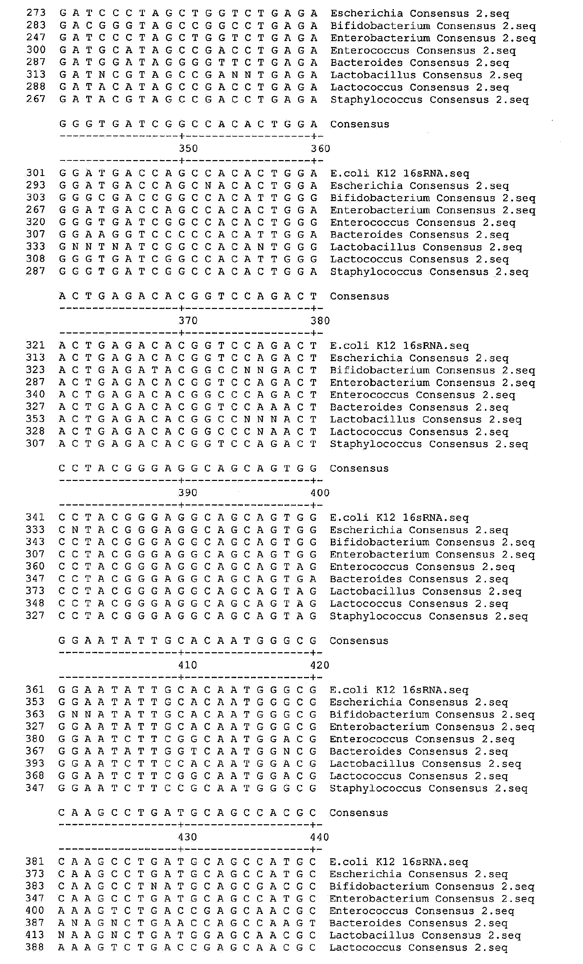

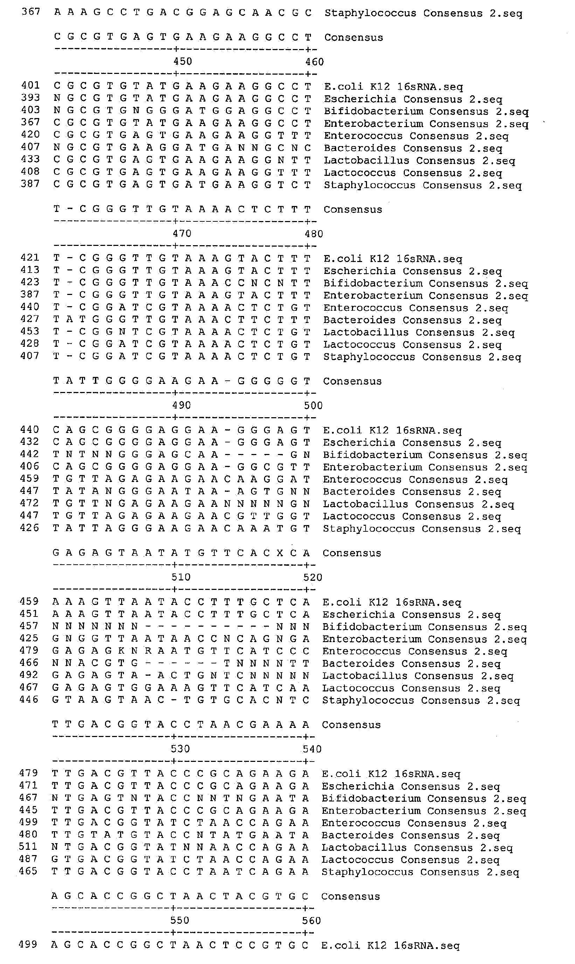

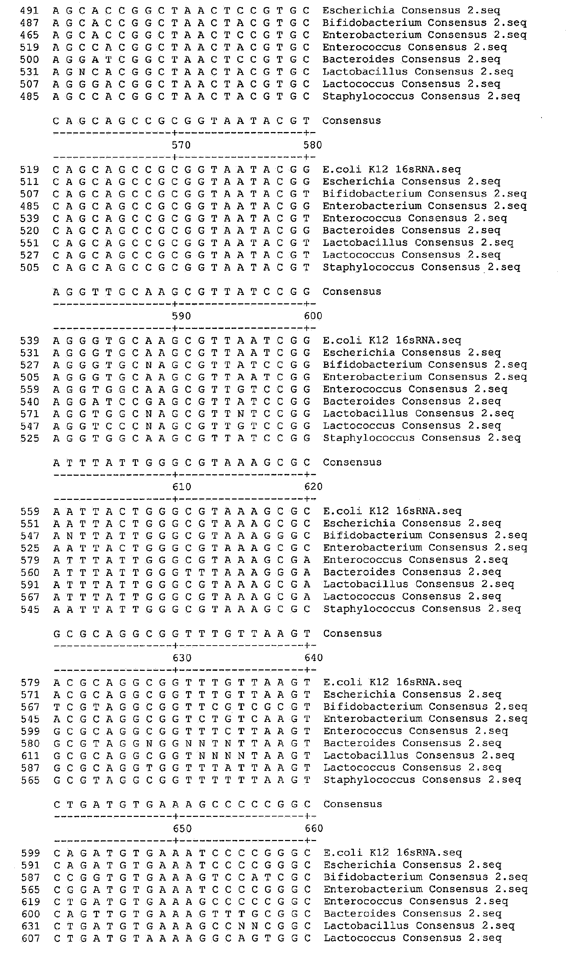

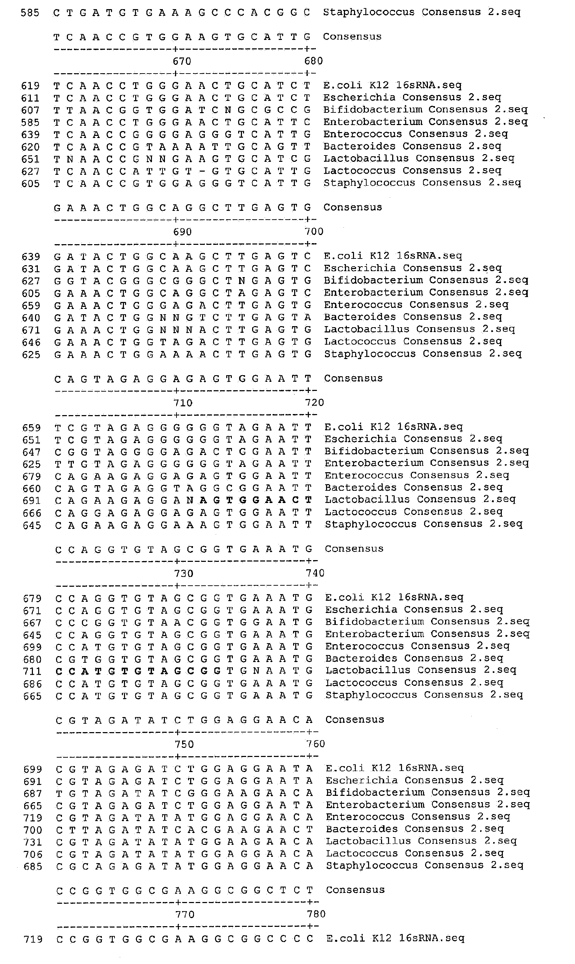

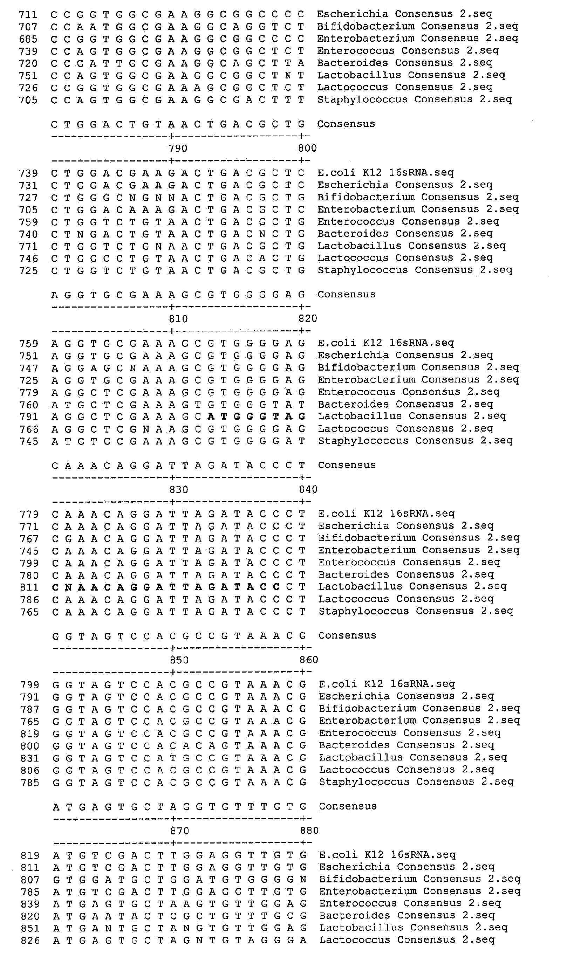

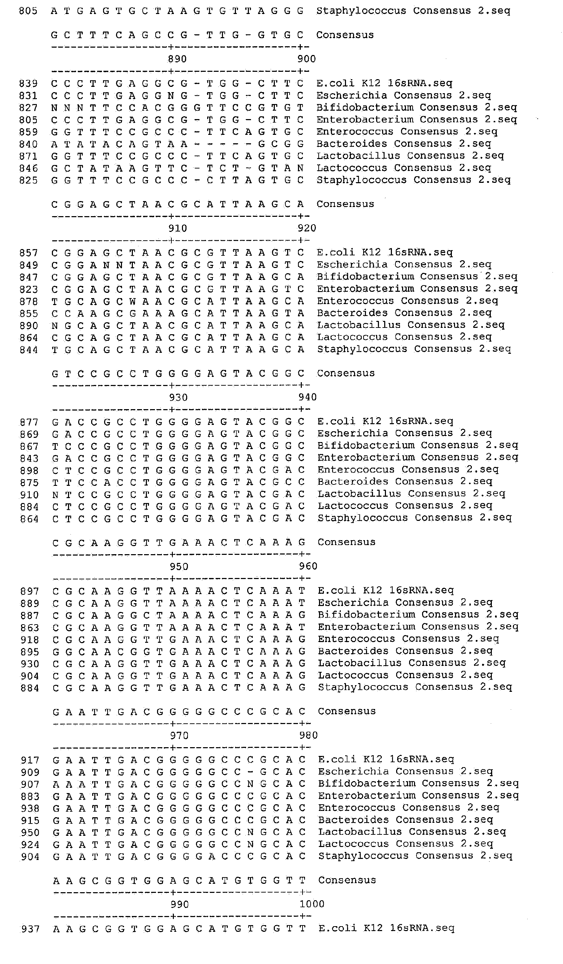

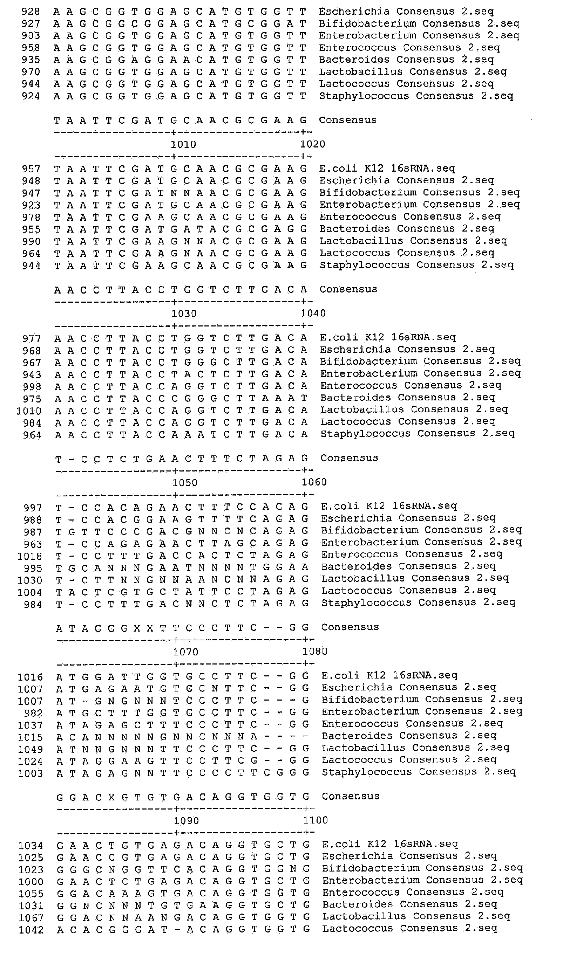

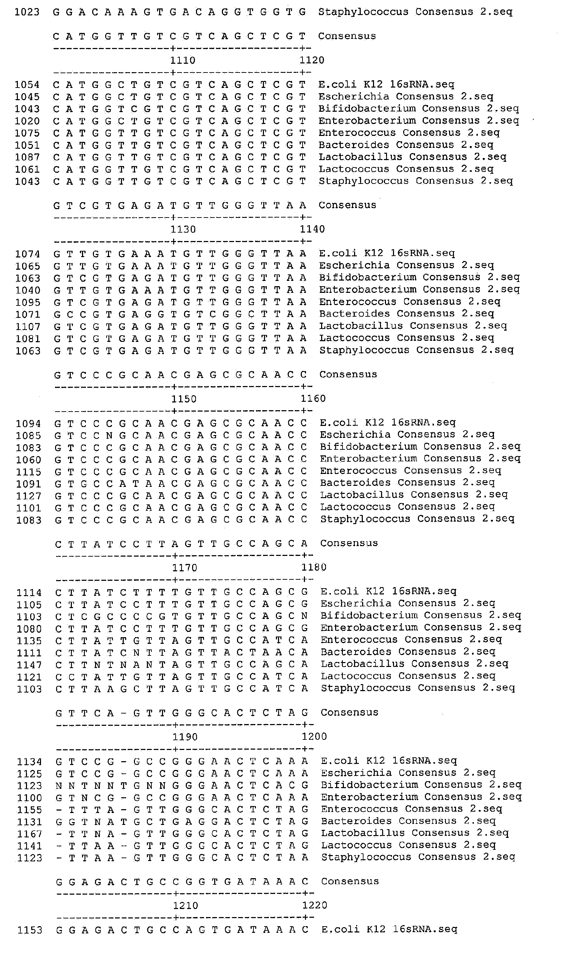

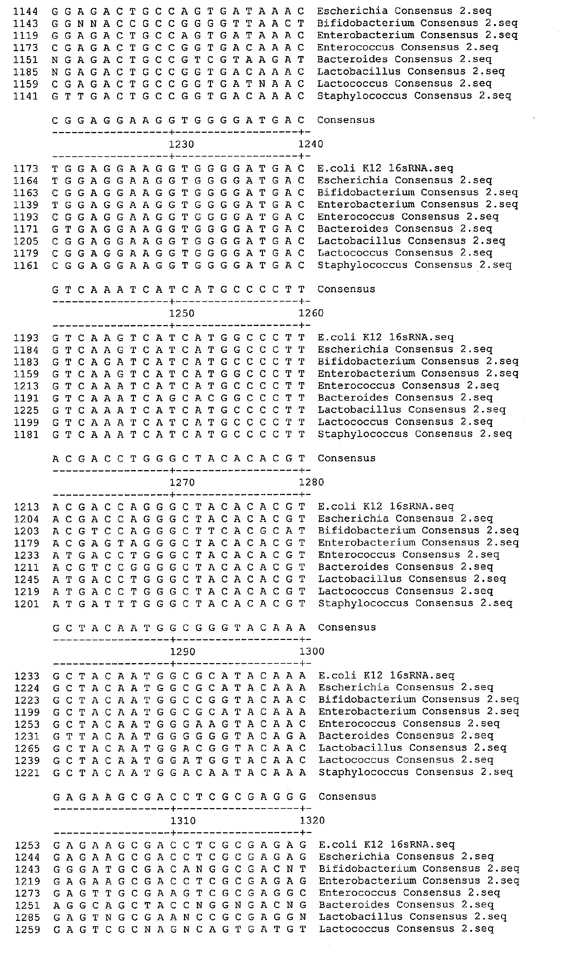

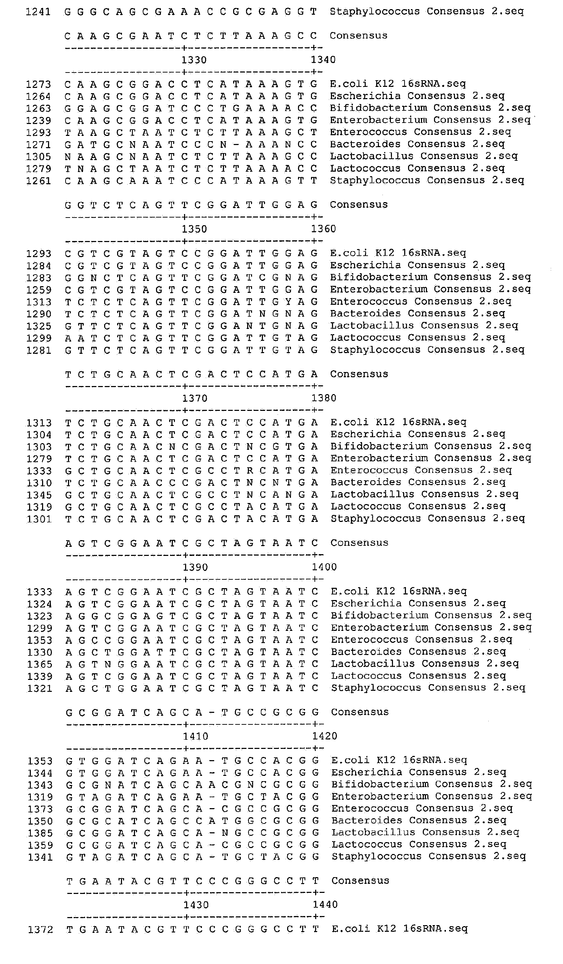

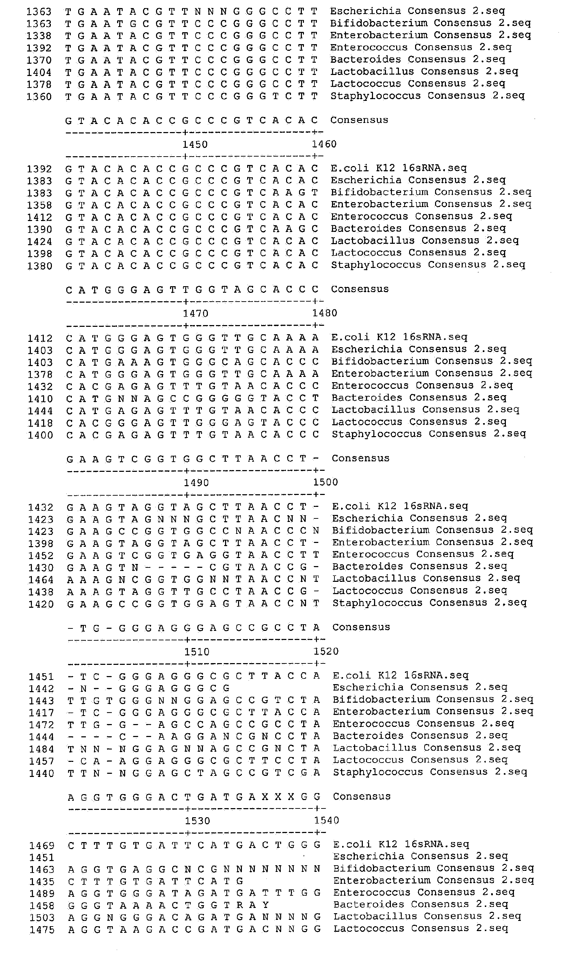

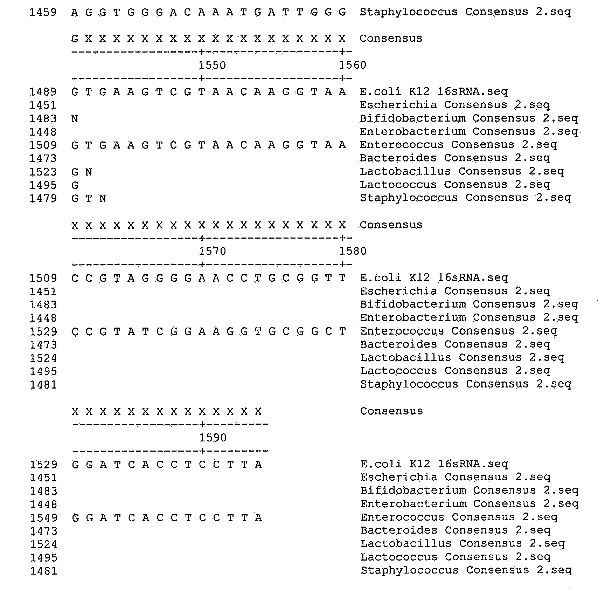

In one aspect the invention provides a method for amplifying nucleic acid of at least one *Lactobacillus* organism, comprising:
- providing said nucleic acid with at least one primer with a length of between 9 and 50 nucleotides, preferably with a length of between 9 and 40 nucleotides, more preferably with a length of between 15 and 25 nucleotides, which primer is capable of specifically hybridizing to a 16S rRNA nucleic acid sequence corresponding to the nucleic acid sequence located from about nucleotide position 702 to about 723 in the consensus Lactobacillus sequence depicted in Figure 1, and/or capable of specifically hybridizing to a 16S rRNA nucleic acid sequence corresponding to the nucleic acid sequence located from about nucleotide position 803 to about 828 in the consensus Lactobacillus sequence depicted in Figure 1, said positions being indicated in Figure 1, and
- performing a nucleic acid amplification reaction.

Ribosomal RNA (rRNA) is the central component of the ribosome, the protein manufacturing machinery of all living cells. The small 30S subunit of prokaryotic ribosomes comprises 16S rRNA and various proteins. The 16S rRNA is important for subunit association and translational accuracy. Figure 1 shows an alignment of (consensus) 16S rRNA nucleic acid sequences of eight bacterial genera. Since the 16S rRNA nucleic acid sequences are highly conserved, the corresponding positions of 16S rRNA nucleic acid sequences in other *Lactobacillus* strains are easily assessed based on homology.

A 16S rRNA nucleic acid sequence is defined herein as any kind of (natural or non-natural) nucleic acid sequence corresponding to a 16S rRNA sequence. A nucleic acid sequence corresponds to a 16S rRNA sequence when said nucleic acid sequence comprises a sequence which is capable of specifically binding to a complementary sequence of said 16S rRNA sequence. A nucleic acid sequence also corresponds to a 16S rRNA sequence when said nucleic acid sequence comprises a sequence which is capable of specifically binding to a 16S rRNA sequence, meaning that said nucleic acid sequence comprises a sequence which is complementary to said 16S rRNA sequence. The term "16S rRNA nucleic acid sequence" for instance encompasses a DNA sequence comprising a sequence which is capable of specifically binding to a 16S rRNA sequence. The term "16S rRNA nucleic acid sequence" also encompasses a DNA sequence comprising a sequence which is identical to a 16S rRNA sequence (except, of course, the fact that DNA comprises thymine instead of uracil). The term "16S rRNA nucleic acid sequence" hence encompasses a 16S rDNA sequence. As used herein, the term "16S rRNA sequence" means any kind of 16S rRNA nucleic acid sequence.

According to the present invention, a primer capable of specifically hybridizing to a 16S rRNA nucleic acid sequence corresponding to the nucleic acid sequence located from about nucleotide position 702 to about 723 in the consensus Lactobacillus sequence depicted in Figure 1, (preferably also capable of specifically hybridizing to a 16S rRNA nucleic acid sequence corresponding to the nucleic acid sequence located from about nucleotide position 712 to about 733 in the general consensus sequence depicted in Figure 1), and/or capable of specifically hybridizing to a 16S rRNA nucleic acid sequence corresponding to the nucleic acid sequence located from about nucleotide position 803 to about 828 in the consensus Lactobacillus sequence depicted in Figure 1 (preferably also capable of specifically hybridizing to a 16S rRNA nucleic acid sequence corresponding to the nucleic acid sequence located from about nucleotide position 813 to about 838 in the general consensus sequence depicted in Figure 1) is particularly suitable for amplifying *Lactobacillus* nucleic acid because such primer is capable of amplifying nucleic acid of a wide variety of *Lactobacillus* strains. Hence, the chance of obtaining a false-negative result (lack of amplification of nucleic acid of a *Lactobacillus* strain present in a sample) is very small. Moreover, a primer according to the invention is highly specific for *Lactobacillus.* The chance of amplifying non-*Lactobacillus* nucleic acid is therefore reduced, as compared to current methods such as the one disclosed in Song et al. (FEMS Microbiology Letters 187 (2000) 167-173).

An amplification reaction is defined herein as a reaction during which a nucleic acid sequence is duplicated at least one time. Preferred amplification reactions comprise (reverse transcriptase)-polymerase chain reaction ((RT)-PCR), nucleic acid sequence based amplification (NASBA), strand displacement amplification (SDA) and transcription mediated amplification (SDA). Methods of performing a nucleic acid amplification reaction are well known in the art. A PCR protocol is for instance described in (Sambrook, J., Fritsch, E.F., and Maniatis, T. 1989. Molecular cloning: a laboratory manual. Cold Spring Harbor laboratory Press, Cold Spring Harbor, N.Y.). (Called herein Sambrook et al (1989)).

A first nucleic acid molecule (such as a primer or a probe) specifically hybridizes to a second nucleic acid molecule if said first nucleic acid molecule comprises a sequence of at least four, preferably at least five, more preferably at least six nucleotides which is complementary to at least part of a sequence of said second nucleic acid molecule. As is well known by the skilled person, adenine (A) is complementary to thymine (T) and uracil (U). Guanine (G) is complementary to cytosine (C). The term "specifically hybridizing" or "specifically binding" of a nucleic acid sequence to another nucleic acid sequence therefore means a binding event resulting from hybridization of complementary nucleic acid sequences. The terms do not encompass nonspecific sticking of nucleic acid molecules, which binding is not due to a complementary sequence. However, the terms encompass a binding event of two nucleic acid molecules under mild conditions, which nucleic acid molecules comprise sequences that are less than 100% but more than 65% complementary to each other. Two nucleic acid sequences that are less than 100% but more than 65% complementary to each other are nevertheless capable of hybridizing under mild conditions such as for instance a low temperature. Each of said two nucleic acid molecules preferably comprises a sequence that is at least 70%, preferably at least 75%, more preferably at least 80%, more preferably at least 85% complementary to a sequence of the other nucleic acid molecule.

If stringent conditions are used, however, two nucleic acid molecules only bind if they comprise a sequence which is at least 85%, preferably at least 90% complementary to a sequence of the other nucleic acid molecule. Stringent conditions are therefore defined herein as conditions where hybridization of nucleic acid molecules only takes place if said nucleic acid molecules comprise a sequence which is at least 85%, preferably at least 90% complementary to a sequence of the other nucleic acid molecule.

Primer and probe sequences as used herein for instance comprise naturally occurring nucleic acids, such as DNA, RNA or a DNA/RNA helix, modified nucleic acid sequences and/or nucleic acid derivatives such as for instance peptide nucleic acid (PNA). Possible bases of primer and probe sequences according to the invention comprise naturally occurring purines and/or pyrimidines, such as adenine, thymine, cytosine, guanine and uracil, as well as modified bases such as for instance inosine.

The length of a primer according to the invention is preferably between 9 and 50 nucleotides, preferably with a length of between 9 and 40 nucleotides, more preferably between 15 and 25 nucleotides. The length of a probe according to the invention is preferably between 10 and 50 nucleotides, more preferably between 13 and 30 nucleotides.

One preferred embodiment provides a primer with a length of between 9 and 50 nucleotides, preferably with a length of between 9 and 40 nucleotides, more preferably with a length of between 15 and 25 nucleotides, comprising a nucleic acid sequence which is at least 70% homologous to at least part of the sequence AGTGGAACTCCATGTGTAGCGG or GGTATCTAATCCTGTTCGCTACCCAT, said part having at least 10 nucleotides. These primers are particularly specific for *Lactobacillus.* Said primers are suitable for amplifying nucleic acid of a wide variety of *Lactobacillus* strains, while amplification of *non-Lactobacillus* nucleic acid barely occurs, if at all. Further provided is therefore a method for amplifying nucleic acid of at least one *Lactobacillus* organism, comprising providing said nucleic acid with a primer with a length of between 9 and 50 nucleotides, preferably with a length of between 9 and 40 nucleotides, more preferably with a length of between 15 and 25 nucleotides, said primer comprising a nucleic acid sequence which is at least 70% homologous to at least part of the sequence AGTGGAACTCCATGTGTAGCGG or GGTATCTAATCCTGTTCGCTACCCAT, said part having at least 10 nucleotides. Preferably said part comprises at least 15 nucleotides, more preferably at least 20 nucleotides. Said primer preferably comprises a nucleic acid sequence which is at least 75%, more preferably at least 80%, more preferably at least 85%, more preferably at least 90%, most preferably at least 95% homologous to at least part of the sequence AGTGGAACTCCATGTGTAGCGG or GGTATCTAATCCTGTTCGCTACCCAT, said part having at least 10 nucleotides. In one preferred embodiment a primer consisting of the sequence AGTGGAACTCCATGTGTAGCGG or GGTATCTAATCCTGTTCGCTACCCAT is used.

In a particularly preferred embodiment, at least two different primers according to the invention are used, wherein one primer is at least 70% homologous to at least part of the sequence AGTGGAACTCCATGTGTAGCGG, said part having at least 10 nucleotides, and another primer is at least 70% homologous to at least part of the sequence GGTATCTAATCCTGTTCGCTACCCAT, said part having at least 10 nucleotides. Preferably a primer pair is used wherein a primer with a length of between 9 and 50 nucleotides, preferably with a length of between 9 and 40 nucleotides, more preferably with a length of between 15 and 25 nucleotides, comprising a nucleic acid sequence which is at least 70%, preferably at least 75%, more preferably at least 80%, more preferably at least 85%, more preferably at least 90%, most preferably at least 95% homologous to at least part of the sequence AGTGGAACTCCATGTGTAGCGG, said part having at least 10 nucleotides, is combined with a primer with a length of between 9 and 50 nucleotides, preferably with a length of between 9 and 40 nucleotides, more preferably with a length of between 15 and 25 nucleotides, comprising a nucleic acid sequence which is at least 70%, preferably at least 75%, more preferably at least 80%, more preferably at least 85%, more preferably at least 90%, most preferably at least 95% homologous to at least part of the sequence GGTATCTAATCCTGTTCGCTACCCAT, said part having at least 10 nucleotides. This combination of primers according to the invention is particularly suitable for specifically amplifying a wide variety of *Lactobacillus* nucleic acid, while at least in part avoiding amplification of *non-Lactobacillus* nucleic acid. In one particularly preferred embodiment a primer consisting of the sequence AGTGGAACTCCATGTGTAGCGG in combination with a primer consisting of the sequence GGTATCTAATCCTGTTCGCTACCCAT is used.

The invention furthermore provides probe sequences which are particularly suitable for detecting, isolating and/or quantifying *Lactobacillus* nucleic acid. Probes according to the invention are at least 70% homologous to at least part of the sequence CGTAGATATATGGAAGAACACCAGT, said part having at least 15 nucleotides. Such probe is capable of specifically binding to nucleic acid of a wide variety of *Lactobacillus* strains. The chance of false-negative results is reduced as compared to currently known methods. Furthermore, false-positive results are reduced as well because a probe according to the invention is highly specific for *Lactobacillus.* One preferred embodiment therefore provides a method according to the invention, further comprising detecting amplified nucleic acid with a probe with a length of between 10 and 50 nucleotides, preferably between 13 and 30 nucleotides, comprising a nucleic acid sequence which is at least 70% homologous to at least part of the sequence CGTAGATATATGGAAGAACACCAGT, said part having at least 15 nucleotides. Preferably, said part comprises at least 20 nucleotides. Said probe preferably comprises a nucleic acid sequence which is at least 75%, more preferably at least 80%, more preferably at least 85%, more preferably at least 90%, most preferably at least 95% homologous to at least part of the sequence CGTAGATATATGGAAGAACACCAGT, said part having at least 15 nucleotides, preferably at least 20 nucleotides. The higher the homology, the more *Lactobacillus* strains are detectable with said probe. In one particularly preferred embodiment a probe consisting of the sequence CGTAGATATATGGAAGAACACCAGT is used.

Methods for screening a sample for the presence of *Lactobacillus* nucleic acid are also herewith provided. One preferred embodiment provides a method for determining whether a sample comprises nucleic acid of at least one *Lactobacillus* organism, comprising:
- administering to nucleic acid of said sample a probe with a length of between 10 and 50 nucleotides, preferably between 13 and 30 nucleotides, comprising a nucleic acid sequence which is at least 70% homologous to at least part of the sequence CGTAGATATATGGAAGAACACCAGT, said part having at least 15 nucleotides, preferably at least 20 nucleotides, and
- determining whether said probe specifically binds nucleic acid of said sample.

Said probe preferably comprises a nucleic acid sequence which is at least 75%, more preferably at least 80%, more preferably at least 85%, more preferably at least 90%, most preferably at least 95% homologous to at least part of the sequence CGTAGATATATGGAAGAACACCAGT, said part having at least 15 nucleotides, preferably at least 20 nucleotides. The higher the homology, the more *Lactobacillus* strains are detectable with said probe. In one particularly preferred embodiment a probe consisting of the sequence CGTAGATATATGGAAGAACACCAGT is used.

In order to detect a low amount of nucleic acid of interest in a sample, a nucleic acid amplification reaction is preferably performed. A preferred embodiment thus provides a method according to the invention, wherein nucleic acid of said sample is subjected to a nucleic acid amplification reaction. In one embodiment nucleic acid is firstly amplified, where after a probe according to the invention is administered to the amplified nucleic acid in order to determine whether *Lactobacillus* nucleic acid is present. Amplified nucleic acid is incubated with at least one probe, unbound probe is preferably washed away and bound probe is visualized, for instance using a visible label and/or an enzyme reaction. These methods are well known and need no further explanation here. Preferably however real time monitoring is performed, for instance using a Taqman or beacon probe. A Taqman probe comprises an oligonucleotide, complementary to a target sequence, which is labeled with a 5'-reporter dye and a 3'-quencher dye. When both dyes are attached to the probe the reporter dye cannot be detected. A TaqMan probe hybridizes to a target nucleic acid sequence. During a nucleic acid amplification reaction the TaqMan probe is hydrolyzed, resulting in separation of the reporter dye and quencher dyes. This, in turn, results in increased fluorescence of the reporter. Increase in fluorescence is monitored over time. Many well known alternative methods for detection of amplified nucleic acid are available in the art

In order to specifically amplify *Lactobacillus* nucleic acid, a primer capable of specifically hybridizing to *Lactobacillus* nucleic acid is preferably used. Said primer is capable of binding a higher number of different *Lactobacillus* nucleic acid sequences, as compared to the number of different *non-Lactobacillus* nucleic acid sequences that said primer is capable of binding to. Such primer is called herein a *Lactobacillus-specific* primer. Said primer is preferably capable of binding less than ten, preferably less than eight, more preferably less than six different *non-Lactobacillus* nucleic acid sequences. Most preferably said primer is only capable of specifically binding to nucleic acid of *Lactobacillus* strains. This way, amplification of nucleic acid of other species is avoided to a large extent. A method according to the invention comprising subjecting nucleic acid of a sample to a nucleic acid amplification reaction using at least one *Lactobacillus-specific* primer is therefore also provided.

As described above, nucleic acid is preferably amplified using at least one *Lactobacillus*-specific primer according to the invention with a length of between 9 and 50 nucleotides, preferably with a length of between 9 and 40 nucleotides, more preferably with a length of between 15 and 25 nucleotides, comprising a nucleic acid sequence which is at least 70% homologous to at least part of the sequence AGTGGAACTCCATGTGTAGCGG or GGTATCTAATCCTGTTCGCTACCCAT, said part having at least 10 nucleotides. In one preferred embodiment a primer consisting of the sequence AGTGGAACTCCATGTGTAGCGG or GGTATCTAATCCTGTTCGCTACCCAT is used. In a particularly preferred embodiment, at least two different primers according to the invention are used, wherein one primer is at least 70% homologous to at least part of the sequence AGTGGAACTCCATGTGTAGCGG, said part having at least 10 nucleotides, and another primer is at least 70% homologous to at least part of the sequence GGTATCTAATCCTGTTCGCTACCCAT, said part having at least 10 nucleotides. This combination of primers according to the invention is particularly suitable for specifically amplifying a wide variety of *Lactobacillus* nucleic acid, while at least in part avoiding amplification of non-*Lactobacillus* nucleic acid. In one particularly preferred embodiment a primer consisting of the sequence AGTGGAACTCCATGTGTAGCGG in combination with a primer consisting of the sequence GGTATCTAATCCTGTTCGCTACCCAT is used.

If a member of the *Lactobacillus* genus is present in a sample, it is particularly well detected with a probe according to the present invention. Such probe is also suitable for isolating nucleic acid of a *Lactobacillus* member. Detection and/or isolation of *Lactobacillus* nucleic acid is for instance performed using a column or dipstick with at least one probe according to the invention. Such column or dipstick is incubated with a sample, where after unbound components are washed away and bound nucleic acid is detected and/or isolated. Various alternative ways of detecting and/or isolating nucleic acid using a given probe sequence are available in the art, which alternative ways are also applicable. Further provided is therefore a method for detecting and/or isolating nucleic acid of at least one *Lactobacillus* organism, comprising administering a probe with a length of between 10 and 50 nucleotides, preferably between 13 and 30 nucleotides, comprising a nucleic acid sequence which is at least 70% homologous to at least part of the sequence CGTAGATATATGGAAGAACACCAGT, said part having at least 15 nucleotides, preferably at least 20 nucleotides, to nucleic acid of at least one *Lactobacillus* organism. Said probe preferably comprises a nucleic acid sequence which is at least 75%, more preferably at least 80%, more preferably at least 85%, more preferably at least 90%, most preferably at least 95% homologous to at least part of the sequence CGTAGATATATGGAAGAACACCAGT, said part having at least 15 nucleotides, preferably at least 20 nucleotides. The higher the homology, the more *Lactobacillus* strains are detectable with said probe. Most preferably, said probe consists of the sequence CGTAGATATATGGAAGAACACCAGT.

For some applications detection of the presence or absence of *Lactobacillus* nucleic acid is sufficient, for instance if contamination of a supposedly sterile sample is examined, if the presence of non-starters in cheese is investigated or if the presence of spoilers in beer is investigated. In other applications however, quantification of the amount of *Lactobacillus* nucleic acid is preferred. For instance, the amount of *Lactobacillus* nucleic acid in a sample derived from an individual's gastrointestinal tract is preferably quantified because a sufficiently high, natural amount of *Lactobacillus* is important for the well-being of individuals whereas a diminished amount of *Lactobacillus* has an influence on the general health status and often coincides with an increase in susceptibility to gastroenterological infections and chronic diseases like colon cancer. A method according to the invention which further comprises quantifying nucleic acid of at least one *Lactobacillus* organism is therefore also provided herewith. Quantitation of nucleic acid is for instance performed using quantitative nucleic acid amplification, which is well known in the art. In one embodiment, known amounts of nucleic acid of interest are amplified and visualised beforehand. The obtained values are plotted in order to obtain a reference curve. Subsequently, an unknown amount of nucleic acid is amplified and visualised, where after the obtained value is compared with said reference curve in order to establish the (original) amount of said nucleic acid of interest. In another embodiment real time quantitative nucleic acid amplification is used with Taqman or beacon probes. According to this embodiment an amplification plot is established in which a fluorescence signal is plotted versus the cycle number. In the initial cycles of a nucleic acid amplification reaction, there is generally little change in the fluorescence signal. This defines the baseline for the amplification plot. An increase in fluorescence above the baseline implies detection of accumulated nucleic acid amplification product. A fixed fluorescence threshold is preferably set above the baseline. According to this embodiment a parameter Ct is defined as the fractional cycle number at which the fluorescence passes the fixed threshold. Quantitation of the amount of target nucleic acid in unknown samples is subsequently preferably accomplished by measuring the Ct and using said standard curve to determine the starting copy number.

Nucleic acid is also quantified in various alternative ways, which are known in the art and need no further explanation.

In one embodiment the presence and/or amount of total *Lactobacillus* nucleic acid content in a sample is investigated. Additionally, or alternatively, nucleic acid of at least one *Lactobacillus* organism is identified. This embodiment is particularly suitable in situations wherein the presence of at least one particular *Lactobacillus* species or strain is investigated, for instance in a food product such as a dairy product. *Lactobacillus* nucleic acid is for instance identified when nucleic acid of an unknown species (for instance present in a food product) is amplified and identified, or when nucleic acid of a pool of *Lactobacilli* (for instance present in faeces) is amplified after which the different species are identified in order to obtain more insight in the different species present.

A method according to the invention is, amongst other things, particularly suitable for investigating the presence and/or amount of *Lactobacillus* species in the gastrointestinal tract. A faeces sample is particularly well suitable for this purpose. Further provided is therefore a method according to the invention, wherein said nucleic acid is obtained from a faeces sample.

Further provided are probes and primers according to the present invention, as well as kits comprising these primers and/or probes, which are particularly suitable for detecting, amplifying, quantifying and/or isolating *Lactobacillus* nucleic acid. A nucleic acid sequence with a length of between 10 and 50 nucleotides, preferably between 13 and 30 nucleotides, comprising a sequence which is at least 70%, preferably at least 75%, more preferably at least 80%, more preferably at least 85%, more preferably at least 90%, most preferably at least 95% homologous to at least part of the sequence CGTAGATATATGGAAGAACACCAGT, said part having at least 15 nucleotides, preferably at least 20 nucleotides, is therefore also herewith provided. Such nucleic acid sequence is particularly suitable for use as a probe. Said nucleic acid preferably consists of the sequence CGTAGATATATGGAAGAACACCAGT. A nucleic acid sequence with a length of between 9 and 50 nucleotides, preferably with a length of between 9 and 40 nucleotides, more preferably with a length of between 15 and 25 nucleotides, comprising a sequence which is at least 70%, preferably at least 75%, more preferably at least 80%, more preferably at least 85%, more preferably at least 90%, most preferably at least 95% homologous to at least part of the sequence AGTGGAACTCCATGTGTAGCGG, said part having at least 10 nucleotides, preferably at least 15 nucleotides, more preferably at least 20 nucleotides, is also herewith provided. Such nucleic acid is particularly suitable for use as a primer for specifically amplifying *Lactobacillus* nucleic acid. Said nucleic acid preferably consists of the sequence AGTGGAACTCCATGTGTAGCGG. The invention furthermore provides a nucleic acid sequence with a length of between 9 and 50 nucleotides, preferably with a length of between 9 and 40 nucleotides, more preferably with a length of between 15 and 25 nucleotides, comprising a sequence which is at least 70%, preferably at least 75%, more preferably at least 80%, more preferably at least 85%, more preferably at least 90%, most preferably at least 95% homologous to at least part of the sequence GGTATCTAATCCTGTTCGCTACCCAT, said part having at least 10 nucleotides, preferably at least 15 nucleotides, more preferably at least 20 nucleotides. Such nucleic acid is also particularly suitable for use as a primer for specifically amplifying *Lactobacillus* nucleic acid. Said nucleic acid preferably consists of the sequence GGTATCTAATCCTGTTCGCTACCCAT.

A particularly preferred embodiment provides a primer pair consisting of two different primers according to the invention. Further provided is therefore a primer pair comprising:
- a primer with a length of between 9 and 50 nucleotides, preferably with a length of between 9 and 40 nucleotides, more preferably with a length of between 15 and 25 nucleotides, comprising a sequence which is at least 70%, preferably at least 75%, more preferably at least 80%, more preferably at least 85%, more preferably at least 90%, most preferably at least 95% homologous to at least part of the sequence AGTGGAACTCCATGTGTAGCGG, said part having at least 10 nucleotides, preferably at least 15 nucleotides, more preferably at least 20 nucleotides ,and
- a primer with a length of between 9 and 50 nucleotides, preferably with a length of between 9 and 40 nucleotides, more preferably with a length of between 15 and 25 nucleotides, comprising a sequence which is at least 70%, preferably at least 75%, more preferably at least 80%, more preferably at least 85%, more preferably at least 90%, most preferably at least 95% homologous to at least part of the sequence GGTATCTAATCCTGTTCGCTACCCAT, said part having at least 10 nucleotides, preferably at least 15 nucleotides, more preferably at least 20 nucleotides.

In one particularly preferred embodiment a primer pair consisting of a primer consisting of the sequence AGTGGAACTCCATGTGTAGCGG, and a primer consisting of the sequence GGTATCTAATCCTGTTCGCTACCCAT, is provided.

The invention furthermore provides a kit for detecting *Lactobacillus* nucleic acid, comprising at least one probe according to the invention. A kit for amplifying *Lactobacillus* nucleic acid comprising at least one primer according to the invention is also provided. Said kit preferably comprises a primer pair according to the invention. A kit comprising at least one probe and at least one primer according to the present invention is also provided. A kit comprising at least one probe and a primer pair according to the present invention is preferred.

In one aspect a method according to the invention is used for testing whether a candidate compound is capable of influencing the amount of *Lactobacillus* in a population. This is for instance useful for identifying, developing and/or testing probiotics and/or prebiotics. Probiotics and prebiotics are useful for improving and/or restoring a normal healthy equilibrium in an individual's digestive tract. Probiotics are live micro-organisms, such as *Lactobacillus* species, that are capable of improving the balance of the intestinal microflora. Probiotic bacterial cultures assist the body's naturally occurring flora within the digestive tract to reestablish themselves. Prebiotics are food ingredients that beneficially affect the host by selectively stimulating the growth and/or the activity of a limited number of bacteria in the colon. In order to monitor the effect of a certain probiotic or prebiotic, or in order to screen a candidate compound for beneficial activity, the effects upon the presence and/or amount of *Lactobacillus* is preferably assessed since Lactobacilli are known to contribute to the well-being of individuals. The effect of a (candidate) probiotic and/or (candidate) prebiotic is preferably assessed by determining the presence and/or amount of Lactobacilli in a sample from an individual that has been provided with said (candidate) probiotic and/or (candidate) prebiotic, using a method according to the present invention. Preferably, the presence and/or amount of *Lactobacillus* in a first sample of an individual is investigated with a method according to the invention before said individual has been provided with a (candidate) probiotic and/or (candidate) prebiotic. Subsequently, the presence and/or amount of *Lactobacillus* in the same kind of sample (called herein a second sample) of said individual is investigated with a method according to the invention, which second sample is obtained after said individual has been provided with said (candidate) probiotic and/or (candidate) prebiotic. A difference in the amount or concentration of *Lactobacillus* in said first and second sample is indicative for an effect of said (candidate) probiotic and/or (candidate) prebiotic. Further provided is therefore a method for determining whether a candidate compound is capable of influencing the amount of *Lactobacillus* in a population, comprising:
- providing said population with said candidate compound, and
- determining whether nucleic acid of at least one *Lactobacillus* organism is present in a sample of said population, using a method according to the invention. Said population preferably comprises a population of the gastrointestinal tract. If *Lactobacillus* nucleic acid is present, nucleic acid of at least one *Lactobacillus* strain is preferably quantified because the amount of *Lactobacillus* is indicative for the health status of an individual. Preferably, all *Lactobacillus* nucleic acid present in said sample is quantified. The presence and/or amount (concentration) of *Lactobacillus* nucleic acid in said sample is preferably compared with the presence and/or amount (concentration) of *Lactobacillus* nucleic acid in a reference sample of said population, obtained before said population had been provided with said (candidate) compound.

If a (candidate) compound appears to be capable of beneficially influencing the amount of *Lactobacillus* in said population, it is preferably selected and/or isolated. A method according to the invention, further comprising selecting and/or isolating a candidate compound capable of influencing the amount of *Lactobacillus* in said population is therefore also provided. Preferably a candidate compound is selected and/or isolated which is capable of increasing the amount of *Lactobacillus* in said population.

Said selected and/or isolated compound is preferably incorporated into a food product in order to improve health. Said selected and/or isolated compound is preferably incorporated into a dairy product. A food product capable of beneficially influencing the amount of *Lactobacillus,* obtainable by a method according to the invention is therefore also herewith provided. Said food product preferably comprises a dairy product.

The invention is further explained in the following examples. These examples do not limit the scope of the invention, but merely serve to clarify the invention.

### Examples

### Example 1

Agarose gel with fragments obtained with primers of Yu-li Song et al (2000). FEMS Microbiology Letters 187 (2000) 167-173

| primers: | |
|---|---|
| Lac2 (prR) | CCTCTTCGCTCGCCGCTACT |
| | |
| Ldel-7 (prF1) | ACAGATGGATGGAGAGCAGA |
| LU-1' (prF2) | ATTGTAGAGCGACCGAGAAG |
| LU-3' (prF3) | AAACCGAGAACACCGCGTT |
| LU-4' (prF4) | CTAGCGGTGCGACTTTGTT |

Strains (in this order with each primer set)
1. *L. delbrueckii* subsp. *Lactis* 202 CR118
2. *L. helveticus* CR134
3. *L. Gefilus* GG CR158
4. *L. acidophilus* CR159
5. *L. bulgaricus* CR181
6. *L. casei* CR355
7. *L. plantarum* M16

### Results:

Ten µl of reaction mixture was composed of 0,5 U Taq DNA polymerase (Amersham Biosciences), 1 µl 10x PCR buffer (Amersham Biosciences), 200 µM of each nucleotide, 0,2 µM of each primer and 2 µl of template DNA. PCR was carried out for 35 cycles of 95 °C for 20 sec, 55°C for 30 sec, and 73°C for 60 sec, followed by an additional extension step of 73°C for 180 sec.

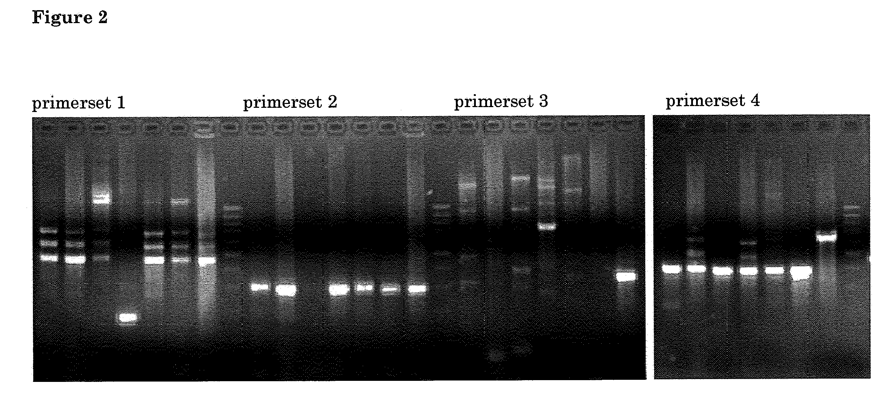

The results are shown in Figure 2: a lot of aspecific PCR products were formed. Hence, the primers of Yu-li Song et al (2000) are not specific enough.

### Example 2

### Three-stage continuous culture system

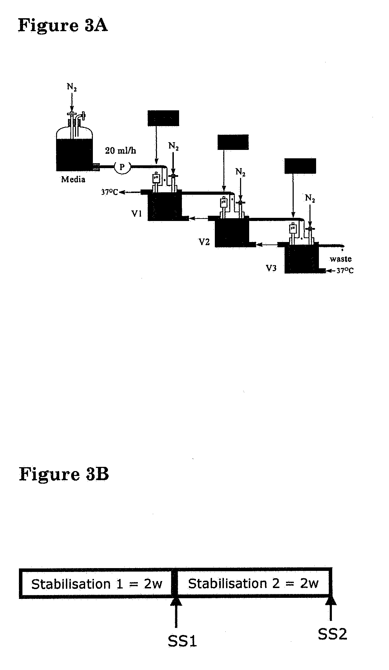

Three autoclaved vessels were connected such that the fermentation media flowed from the media vessel through vessel 1 (volume 280 ml), vessel 2 and vessel 3 (300 ml each) to the waste bottle. See Figure 3A.

Vessels were connected to pH Controller Models 260 (Electrolab) and culture pH maintained at pH 5.5 -5.8 (vessel 1), 6.2 -6.5 (vessel 2), and 6.8 -7.1 (vessel 3). The autoclaved medium was connected to vessel 1 via a pump. Media and fermentation vessels were constantly stirred and kept at 37°C and anaerobic by a constant nitrogen flow.

After filling half of the vessels with medium, roughly the same amount of a freshly prepared faecal slurry was added to each vessel. Three healthy volunteers who had not used antibiotics in the previous three months donated stool samples. Within 30 min following defecation, faecal slurries were prepared from 1 part freshly voided faecal sample and 9 parts pre-reduced phosphate-buffered saline (PBS) (10 % w/v).

The media pump was turned off, and the inoculated vessels incubated without flow for one day. After 24 hours, the medium pump was started again at a flow rate of ca. 20 mL per hour to simulate a retention time of about 44 hours. After two weeks, when an equilibrium or steady state was reached, the carbohydrates in the media were proportionally replaced by the testing substrate. The medium of the last 2 weeks contained 1% substrate. At the time points 0, days 12, 13, 14, 20/21 and 26, 27, 28 samples were taken. Steady state 1 and 2 is calculated as the average of time point 12, 13 and 14, and 26, 27 and 28. See Figure 3B.

For the isolation of bacterial DNA from faecal samples, the QIAamp DNA Stool Mini Kit from QIAgen was used.

16s RNA DNA was amplified by PCR with oligonucleotide prF21bifido and prR21bifido (Bifidobacterium) and with a Lactobacilli-specific primer set.

### Bifidobacteria-specific primer set (prF21bifido and prR21bifido)

*prF21bifido*: a primer consisting of the sequence
CCTTACCTGGGCTTGACATGT
*prR2lbifido:* a primer consisting of the sequence
GACGTAAGGGGCATGATGATC.

### Lactobacilli-specific primer set:

A primer consisting of the sequence AGTGGAACTCCATGTGTAGCGG in combination with a primer consisting of the sequence
GGTATCTAATCCTGTTCGCTACCCAT.

### Quantitative PCR was used to determine Bifidobacteria and Lactobacilli:

The 16 s RNA was amplified by PCR with oligonucleotides prF21bifido and prR21bifido. Twenty-five µl of reaction mixture was composed of 12,5 µl 2x Universal PCR Master Mix (TaqMan, Applied Biosystems), 0,1 µM of each primer, 0,25 µM probe and 5 µl of template DNA. PCR was carried out for 40 cycles of 95°C for 15 sec and 60° C for 60 sec.

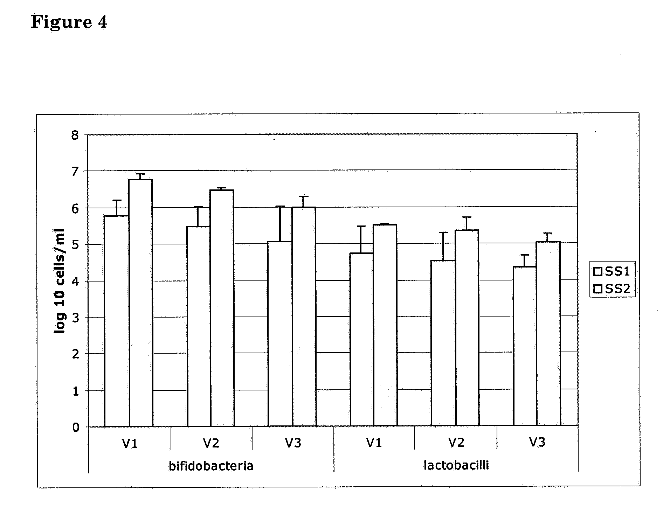

As shown in Figure 4, the addition of Vivinal GOS resulted in significant higher numbers of Bifidobacteria and Lactobacilli (i.e. comparison of steady state 2 (SS2) versus SS1, after correction for donor and vessel and/or substrate).

### Example 3

16s RNA DNA was amplified by PCR with the following Lactobacilli-specific primer set:

### Lactobacilli-specific primer set:

A primer consisting of the sequence AGTGGAACTCCATGTGTAGCGG in combination with a primer consisting of the sequence
GGTATCTAATCCTGTTCGCTACCCAT.

Reagents and reaction conditions:
Tm 55oC 0,5 pmol/ul primer
MgC12 2.5 mM (10x dilution from a stock solution)

- 425 ul Universal Mastermix 25x cycle
- 85 ul forward
   primer
- 85 ul reverse
   primer
- 85 ul 25 mM MgCl2 4.0 mM MgCl2
- 85 ul demi
- 5 ul template (~ 5 ng.ul)

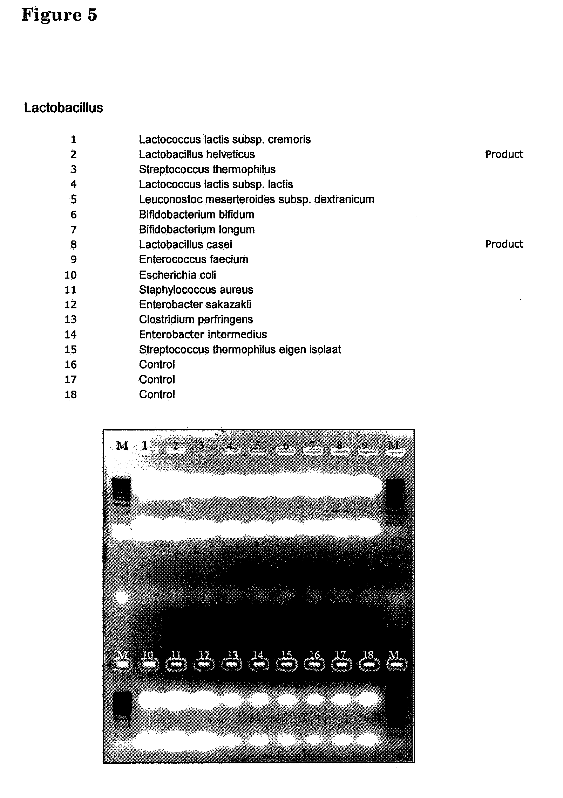

The results are shown in Figure 5.

It is shown that only *Lactobacillus* nucleic acid is amplified. Both *Lactobacillus* strains are amplified. The primers are thus specific for *Lactobacillus* but not too specific. False positive results and false negative results are avoided with these primers.

### Brief description of the drawings

**Figure 1****.** Alignment of 16s rRNA sequences. The 16s rRNA sequence of E. coli K12 represents nucleotide position 223771 - 225312 of the complete genome. Entry: NC_000913 GeneID:944897 16S ribosomal RNA E.coli K12 (Blattner,F.R., Plunkett,G. III, Bloch,C.A., Perna,N.T., Burland,V., Riley,M., Collado-Vides,J., Glasner,J.D., Rode,C.K., Mayhew,G.F., Gregor,J., Davis,N.W., Kirkpatrick,H.A., Goeden,M.A., Rose,D.J., Mau,B. and Shao,Y. (1997). The complete genome sequence of Escherichia coli K-12. Science 277 (5331), 1453-1474).
   Primer sequences are indicated in bold.
   Primer 1 is capable of specifically binding to positions 712-733 of the general consensus sequence and positions 702-723 of the Lactobacillus consensus sequence.
   Primer 2 is capable of specifically binding to positions 813-838 of the general consensus sequence and positions 803-828 of the Lactobacillus consensus sequence.
**Figure 2****.** Results Example 1
**Figure 3****.** Experimental set-up of Example 2
**Figure 4****.** Microbiota in vessels 1, 2, and 3 of the three-stage continuous culture system before (SS1) and 2 weeks after addition of J-GOS (SS2)
**Figure 5****.** Results Example 3

### References

Sambrook, J., Fritsch, E.F., and Maniatis, T. 1989. Molecular cloning: a laboratory manual. Cold Spring Harbor laboratory Press, Cold Spring Harbor, N.Y.). (Called herein Sambrook et al (1989).
Song et al. FEMS Microbiology Letters 187 (2000) 167-173

## Claims

1. A method for amplifying nucleic acid of at least one *Lactobacillus* organism, comprising:
- providing said nucleic acid with at least one primer with a length of between 9 and 50 nucleotides, preferably with a length of between 9 and 40 nucleotides, more preferably with a length of between 15 and 25 nucleotides, which primer is capable of specifically hybridizing to a 16S rRNA nucleic acid sequence corresponding to the nucleic acid sequence located from about nucleotide position 702 to about 723 in the consensus Lactobacillus sequence depicted in Figure 1, and/or capable of specifically hybridizing to a 16S rRNA nucleic acid sequence corresponding to the nucleic acid sequence located from about nucleotide position 803 to about 828 in the consensus Lactobacillus sequence depicted in Figure 1, said positions being indicated in Figure 1, and
- performing a nucleic acid amplification reaction.

2. A method according to claim 1, comprising providing said nucleic acid with a primer with a length of between 9 and 50 nucleotides, preferably with a length of between 9 and 40 nucleotides, more preferably with a length of between 15 and 25 nucleotides, comprising a nucleic acid sequence which is at least 70% homologous to at least part of the sequence
AGTGGAACTCCATGTGTAGCGG or
GGTATCTAATCCTGTTCGCTACCCAT, said part having at least 10 nucleotides.

3. A method according to claim 1 or 2, further comprising detecting amplified nucleic acid with a probe with a length of between 10 and 50 nucleotides, preferably between 13 and 30 nucleotides, comprising a nucleic acid sequence which is at least 70% homologous to at least part of the sequence CGTAGATATATGGAAGAACACCAGT, said part having at least 15 nucleotides.

4. A method for determining whether a sample comprises nucleic acid of at least one *Lactobacillus* organism, comprising:
- administering to nucleic acid of said sample a probe with a length of between 10 and 50 nucleotides, preferably between 13 and 30 nucleotides, comprising a nucleic acid sequence which is at least 70% homologous to at least part of the sequence CGTAGATATATGGAAGAACACCAGT, said part having at least 15 nucleotides, and
- determining whether said probe specifically binds nucleic acid of said sample.

5. A method according to claim 4, wherein nucleic acid of said sample is subjected to a nucleic acid amplification reaction.

6. A method according to claim 5, comprising providing said nucleic acid with a primer with a length of between 9 and 50 nucleotides, preferably with a length of between 9 and 40 nucleotides, more preferably with a length of between 15 and 25 nucleotides, comprising a nucleic acid sequence which is at least 70% homologous to at least part of the sequence
AGTGGAACTCCATGTGTAGCGG or
GGTATCTAATCCTGTTCGCTACCCAT, said part having at least 10 nucleotides.

7. A method for detecting and/or isolating nucleic acid of at least one *Lactobacillus* organism, comprising administering a probe with a length of between 10 and 50 nucleotides, preferably between 13 and 30 nucleotides, comprising a nucleic acid sequence which is at least 70% homologous to at least part of the sequence CGTAGATATATGGAAGAACACCAGT, said part having at least 15 nucleotides, to nucleic acid of at least one *Lactobacillus* organism.

8. A method according to any one of claims 1-7, further comprising quantifying nucleic acid of at least one *Lactobacillus* organism.

9. A method according to any one of claims 1-8, further comprising identifying nucleic acid of at least one *Lactobacillus* organism.

10. A method according to any one of claims 1-9, wherein said nucleic acid is obtained from a faeces sample.

11. A nucleic acid sequence with a length of between 10 and 50 nucleotides, preferably between 13 and 30 nucleotides, comprising a sequence which is at least 70% homologous to at least part of the sequence CGTAGATATATGGAAGAACACCAGT, said part having at least 15 nucleotides.

12. A nucleic acid sequence with a length of between 9 and 50 nucleotides, preferably with a length of between 9 and 40 nucleotides, more preferably with a length of between 15 and 25 nucleotides, comprising a sequence which is at least 70% homologous to at least part of the sequence AGTGGAACTCCATGTGTAGCGG, said part having at least 10 nucleotides.

13. A nucleic acid sequence with a length of between 9 and 50 nucleotides, preferably with a length of between 9 and 40 nucleotides, more preferably with a length of between 15 and 25 nucleotides, comprising a sequence which is at least 70% homologous to at least part of the sequence GGTATCTAATCCTGTTCGCTACCCAT, said part having at least 10 nucleotides.

14. A primer pair comprising a primer consisting of a nucleic acid sequence according to claim 12 and a primer consisting of a nucleic acid sequence according to claim 13.

15. A kit for detecting *Lactobacillus* nucleic acid, comprising a probe with a length of between 10 and 50 nucleotides, preferably between 13 and 30 nucleotides, comprising a nucleic acid sequence which is at least 70% homologous to at least part of the sequence CGTAGATATATGGAAGAACACCAGT, said part having at least 15 nucleotides.

16. A kit for amplifying *Lactobacillus* nucleic acid, comprising a nucleic acid sequence with a length of between 9 and 50 nucleotides, preferably with a length of between 9 and 40 nucleotides, more preferably with a length of between 15 and 25 nucleotides, comprising a sequence which is at least 70% homologous to at least part of the sequence
AGTGGAACTCCATGTGTAGCGG or
GGTATCTAATCCTGTTCGCTACCCAT, said part having at least 10 nucleotides.

17. A method for determining whether a candidate compound is capable of influencing the amount of *Lactobacillus* in a population, comprising:
- providing said population with said candidate compound, and
- determining whether nucleic acid of at least one *Lactobacillus* organism is present in a sample of said population, using a method according to any one of claims 1-10.

18. A method according to claim 17, further comprising quantifying nucleic acid of at least one *Lactobacillus* strain.

19. A method according to claim 17 or 18, wherein said population comprises a population of the gastrointestinal tract.

20. A method according to any one of claims 17-19, further comprising selecting and/or isolating a candidate compound capable of influencing the amount of *Lactobacillus* in said population.

21. A method according to claim 20, further incorporating said selected and/or isolated compound into a food product.
